# EUROPÄISCHE PATENTSCHRIFT

(11) **EP 0 700 899 B1**
(45) Veröffentlichungstag und Bekanntmachung des Hinweises auf die Patenterteilung: **31.03.1999**
(21) Anmeldenummer: 95113847.8
(22) Anmeldetag: 04.09.1995
(51) Int. Cl.: C07C 279/22, A61K 31/155

(54) **Substituierte Benzoylguanidine, Verfahren zu ihrer Herstellung, ihre Verwendung als Medikament oder Diagnostikum sowie sie enthaltendes Medikament**
Substituted benzoylguanidines, a process for their preparation, their use as drug or diagnostic agents as well as medicaments containing them
Benzoylguanidines substituées, un procédé pour leur préparation, leur emploi comme médicaments ou agent diagnostique ainsi que des médicaments les contenant

(30) Priorität: 09.09.1994 DE 4432101
(43) Veröffentlichungstag der Anmeldung: 13.03.1996
(73) Patentinhaber: HOECHST AKTIENGESELLSCHAFT, 65929 Frankfurt am Main (DE)
(72) Erfinder: Kleemann, Heinz-Werner, Dr., D-65474 Bischofsheim (DE); Lang, Hans-Jochen, Dr., D-65719 Hofheim (DE); Schwark, Jan-Robert, Dr., D-65929 Frankfurt (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 556 672
- EP-A- 0 589 336
- EP-A- 0 627 413
- CA-A- 2 099 445
- DE-A- 3 502 629

## Beschreibung

Die Erfindung betrifft Benzoylguanidine der Formel I 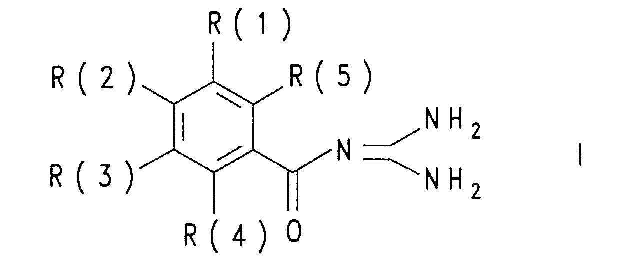 worin bedeuten:
einer der drei Substituenten R(1), R(2) und R(3)
-Y-4-[(CH₂)ₖ-CHR(7)-(C = O)R(8)]-Phenyl,
-Y-3-[(CH₂)ₖ-CHR(7)-(C = O)R(8)]-Phenyl oder
-Y-2-[(CH₂)ₖ-CHR(7)-(C = O)R(8)]-Phenyl,
wobei das Phenyl jeweils unsubstituiert oder substituiert ist mit 1 - 2 Substituenten aus der Gruppe F, Cl, -CF₃, Methyl, Hydroxy, Methoxy, oder -NR(37)R(38); R(37) und R(38)
unabhängig voneinander Wasserstoff oder -CH₃;
- Y: eine Bindung, Sauerstoff, -S- oder -NR(9); R(9) Wasserstoff oder -(C₁-C₄)-Alkyl;
- R(7): -OR(10) oder -NR(10)R(11);
R(10) und R(11)
unabhängig voneinander Wasserstoff, -(C₁-C₈)-Alkyl, -(C₁-C₈)-Alkanoyl, -(C₁-C₈)-Alkoxycarbonyl, Benzyl, Benzyloxycarbonyl;
oder
R(10) Trityl;
- R(8): -OR(12) oder -NR(12)R(13);
R(12) und R(13)
unabhängig voneinander Wasserstoff, -(C₁-C₈)-Alkyl oder Benzyl;
k Null, 1, 2, 3 oder 4;
und die jeweils anderen Reste R(1), R(2) und R(3)
unabhängig voneinander -(C₁-C₈)-Alkyl, -(C₂-C₈)-Alkenyl oder -(CH₂)ₘR(14);
- m: Null, 1 oder 2;
- R(14): -(C₃-C₈)-Cycloalkyl oder Phenyl, welches nicht substituiert oder substituiert ist mit 1 - 3 Substituenten ausgewählt aus der Gruppe bestehend aus F, Cl, -CF₃, Methyl, Methoxy und -NR(15)R(16); R(15) und R(16)
Wasserstoff oder -CH₃;
oder
die jeweils anderen Reste R(1), R(2) und R(3)
unabhängig voneinander R(18)R(19)N-(C = =Y')-NH-SO₂-;
- Y': Sauerstoff, -S- oder -N-R(20);
R(18) und R(19)
unabhängig voneinander Wasserstoff, -(C₁-C₈)-Alkyl, -(C₃-C₆)-
Alkenyl oder -(CH₂)ₜ-R(21);
- t: Null, 1, 2, 3 oder 4;
- R(21): -(C₅-C₇)-Cycloalkyl oder Phenyl, welches unsubstituiert ist oder substituiert ist mit 1 - 3 Substituenten ausgewählt aus der Gruppe bestehend aus F, Cl, -CF₃, Methoxy und -(C₁-C₄)-Alkyl;
oder
R(18) und R(19)
gemeinsam 4 oder 5 Methylengruppen, von denen eine CH₂-Gruppe durch Sauerstoff, -S-, -NH-, -N-CH₃ oder -N-Benzyl ersetzt sein kann;
R(20)
wie R(18) definiert oder Amidin;
oder
die jeweils anderen Reste R(1), R(2) und R(3)
unabhängig voneinander Wasserstoff, F, Cl, Br, I, -C≡N, X-(CH₂)ₚ-(C_{q}F_{2q+1}), R(22)-SOᵤ-, R(23)R(24)N-CO-, R(25)-CO- oder
R(26)R(27)N-SO₂-, wobei die Perfluoralkylgruppe geradkettig oder verzweigt ist;
- X: eine Bindung, Sauerstoff, -S- oder -NR(28);
- u: Null, 1 oder 2;
- p: Null, 1 oder 2;
- q: 1, 2, 3, 4, 5 oder 6;
R(22), R(23), R(25) und R(26)
unabhängig voneinander -(C₁-C₈)-Alkyl, (C₃-C₆)-Alkenyl, -(CH₂)ₙ-R(29) oder -CF₃;
- n: Null, 1, 2, 3 oder 4;
- R(28): Wasserstoff oder -(C₁-C₃)-Alkyl;
- R(29): -(C₃-C₇)-Cycloalkyl oder Phenyl,
welches nicht substituiert ist oder substituiert mit 1 - 3 Substituenten ausgewählt aus der Gruppe bestehend aus F, Cl, -CF₃, Methyl, Methoxy und -NR(30)R(31);
R(30) und R(31)
Wasserstoff oder -(C₁-C₄)-Alkyl;
oder
R(23), R(25) und R(26)
Wasserstoff;
R(24) und R(27)
unabhängig voneinander Wasserstoff oder -(C₁-C₄)-Alkyl;
oder
R(23) und R(24) sowie R(26) und R(27)
gemeinsam 4 oder 5 Methylengruppen, von denen eine CH₂-Gruppe durch Sauerstoff, -S-, -NH-, - N-CH₃ oder -N-Benzyl ersetzt sein kann;
oder
die jeweils anderen Reste R(1), R(2) und R(3)
unabhängig voneinander -OR(35) oder -NR(35)R(36);
R(35) und R(36)
unabhängig voneinander Wasserstoff oder -(C₁-C₆)-Alkyl;
oder
R(35) und R(36)
gemeinsam 4 - 7 Methylengruppen, von denen eine CH₂-Gruppe durch Sauerstoff, -S-, -NH-, -N-CH₃ oder -N-Benzyl ersetzt sein kann;
R(4) und R(5)
unabhängig voneinander Wasserstoff, -(C₁-C₄)-Alkyl, F, Cl, -OR(32), - NR(33)R(34) oder -CᵣF₂ᵣ₊₁;
R(32), R(33) und R(34)
unabhängig voneinander Wasserstoff oder -(C₁-C₃)-Alkyl;
- r: 1, 2, 3 oder 4;
sowie deren pharmazeutisch verträgliche Salze.

Bevorzugt sind Verbindungen der Formel I, in denen bedeuten:
- R(1): -(C₁-C₄)-Alkyl, -(C₂-C₄)-Alkenyl oder -(CH₂)ₘR(14);
m Null, 1 oder 2;
R(14) -(C₅-C₆)-Cycloalkyl oder Phenyl,
welches nicht substituiert oder substituiert ist mit 1 - 2 Substituenten ausgewählt aus der Gruppe bestehend aus F, Cl, -CF₃, Methyl, Methoxy und -NR(15)R(16); R(15) und R(16)
unabhängig voneinander Wasserstoff oder -CH₃;
oder
- R(1): R(18)R(19)N-(C=Y')- NH-SO₂-;
Y' Sauerstoff, -S- oder -N-R(20); R(18) und R(19)
unabhängig voneinander Wasserstoff, -(C₁-C₄)-Alkyl, -(C₃-C₄)-Alkenyl oder -(CH₂)ₜ-R(21);
t Null, 1 oder 2;
R(21)
-(C₅-C₆)-Cycloalkyl oder Phenyl, welches unsubstituiert ist oder substituiert ist mit 1 - 2 Substituenten ausgewählt aus der Gruppe bestehend aus F, Cl, -CF₃, Methoxy und Methyl; oder
R(18) und R(19)
gemeinsam 4 oder 5 Methylengruppen, von denen eine CH₂-Gruppe durch Sauerstoff, -S-, -NH-, -N-CH₃ oder -N-Benzyl ersetzt sein kann;
R(20) wie R(18) definiert oder Amidin;
oder
- R(1): Wasserstoff, F, Cl, Br, I, -C≡N, R(22)-SO₂-, R(23)R(24)N-CO-, R(25)-CO-oder R(26)R(27)N-SO₂-;
R(22), R(23), R(25) und R(26)
unabhängig voneinander -(C₁-C₄)-Alkyl, -(C₃-C₄)-Alkenyl, -(CH₂)ₙ-R(29) oder -CF₃;
n Null, 1 oder 2;
R(29) -(C₅-C₆)-Cycloalkyl oder Phenyl, welches nicht substituiert ist oder substituiert mit 1 - 2 Substituenten ausgewählt aus der Gruppe bestehend aus F, Cl, -CF₃, Methyl, Methoxy und -NR(30)R(31);
R(30) und R(31)
unabhängig voneinander Wasserstoff oder Methyl;
oder
R(23), R(25) und R(26)
Wasserstoff;
R(24) und R(27)
unabhängig voneinander Wasserstoff oder Methyl;
oder
R(23) und R(24) sowie R(26) und R(27)
gemeinsam 4 oder 5 Methylengruppen, von denen eine CH₂-Gruppe durch Sauerstoff, -S-, -NH-, -N-CH₃ oder -N-Benzyl ersetzt sein kann;
oder
- R(1): -OR(35) oder -NR(35)R(36);
R(35) und R(36)
unabhängig voneinander Wasserstoff oder -(C₁-C₄)-Alkyl;
oder
R(35) und R(36)
gemeinsam 4 - 5 Methylengruppen, von denen eine CH₂-Gruppe durch Sauerstoff, -S-, -NH-, -N-CH₃ oder -N-Benzyl ersetzt sein kann;
einer der Substituenten R(2) oder R(3)
Y-4-[(CH₂)ₖ-CHR(7)-(C = O)R(8)]-Phenyl,
-Y-3-[(CH₂)ₖ-CHR(7)-(C = O)R(8)]-Phenyl oder
-Y-2-[(CH₂)ₖ-CHR(7)-(C = O)R(8)]-Phenyl,
wobei das Phenyl jeweils unsubstituiert oder substituiert ist mit 1 - 2 Substituenten aus der Gruppe bestehend aus F, Cl, -CF₃, Methyl, Hydroxy, Methoxy, oder -NR(37)R(38);
R(37) und R(38)
unabhängig voneinander Wasserstoff oder -CH₃;
- Y: eine Bindung, Sauerstoff, -S- oder -NR(9);
R(9) Wasserstoff oder Methyl;
R(7) -OR(10) oder -NR(10)R(11);
R(10) und R(11)
unabhängig voneinander Wasserstoff, -(C₁-C₅)-Alkyl, -(C₁-C₅)-Alkanoyl, -(C₁-C₄)-Alkoxycarbonyl, Benzyl oder Benzyloxycarbonyl;
oder
R(10) Trityl;
R(8) -OR(12) oder -NR(12)R(13);
R(12) und R(13)
unabhängig voneinander Wasserstoff, -(C₁-C₄)-Alkyl oder Benzyl;
k Null, 1 oder 2;
und der jeweils andere Substituent R(2) oder R(3)
-(C₁-C₄)-Alkyl, Wasserstoff, F, Cl, Br oder 1;
R(4) und R(5)
unabhängig voneinander Wasserstoff, Methyl, F, Cl, -OR(32), - NR(33)R(34) oder -CF₃;
R(32), R(33) und R(34)
unabhängig voneinander Wasserstoff oder Methyl;
sowie deren pharmazeutisch verträgliche Salze.

Besonders bevorzugt sind Verbindungen der Formel I, in denen bedeuten:
- R(1): -(C₁-C₄)-Alkyl, -(C₂-C₄)-Alkenyl oder -(CH₂)ₘR(14);
m Null, 1 oder 2;
R(14) -(C₅-C₆)-Cycloalkyl oder Phenyl, welches nicht substituiert oder substituiert ist mit 1 - 2 Substituenten ausgewählt aus der Gruppe bestehend aus F, Cl, -CF₃, Methyl, Methoxy und -NR(15)R(16);
R(15) und R(16)
unabhängig voneinander Wasserstoff oder -CH₃;
oder
- R(1): Wasserstoff, F, Cl, Br, I, -C≡N, R(22)-SO₂-, R(23)R(24)N-CO-, R(25)-CO-
oder R(26)R(27)N-SO₂-;
R(22), R(23), R(25) und R(26)
unabhängig voneinander Methyl oder -CF₃,
oder
R(23), R(25) und R(26)
Wasserstoff;
R(24) und R(27)
unabhängig voneinander Wasserstoff oder Methyl;
oder
- R(1): -OR(35) oder -NR(35)R(36);
R(35) und R(36)
unabhängig voneinander Wasserstoff oder -(C₁-C₄)-Alkyl;
oder
R(35) und R(36)
gemeinsam 4 - 5 Methylengruppen, von denen eine CH₂-Gruppe durch Sauerstoff, -S-, -NH-, -N-CH₃ oder -N-Benzyl ersetzt sein kann;
einer der Substituenten R(2) und R(3)
-Y-4-[(CH₂)ₖ-CHR(7)-(C = O)R(8)]-Phenyl,
-Y-3-[(CH₂)ₖ-CHR(7)-(C=O)R(8)]-Phenyl oder
-Y-2-[(CH₂)ₖCHR(7)-(C = O)R(8)]-Phenyl,
wobei das Phenyl jeweils unsubstituiert oder substituiert ist mit einem Substituenten ausgewählt aus der Gruppe bestehend aus F, Cl, -CF₃, Methyl, Hydroxy, Methoxy und -NR(37)R(38);
R(37) und R(38)
unabhängig voneinander Wasserstoff oder -CH₃;
- Y: eine Bindung, Sauerstoff, -S- oder -NR(9);
- R(9): Wasserstoff oder Methyl;

- R(7): -NR(10)R(11);
R(10) und R(11)
unabhängig voneinander Wasserstoff, -(C₁-C₄)-Alkyl,-(C₁-C₅)-Alkanoyl, -(C₁-C₄)-Alkoxycarbonyl, Benzyl oder Benzyloxycarbonyl;
oder
R(10) Trityl;
- R(8): -OR(12) oder -NR(12)R(13);
R(12) und R(13)
unabhängig voneinander Wasserstoff, -(C₁-C₄)-Alkyl oder Benzyl;
und der jeweils andere Substituent R(2) und R(3)
unabhängig voneinander -(C₁-C₄)-Alkyl, Wasserstoff, F oder Cl;
R(4) und R(5)
unabhängig voneinander Wasserstoff, Methyl, F, Cl, -OR(32), -NR(33)R(34) oder -CF₃;
R(32), R(33) und R(34)
unabhängig voneinander Wasserstoff oder Methyl;
sowie deren pharmazeutisch verträgliche Salze.

Enthält einer der Substituenten R(1) bis R(5) ein oder mehrere Asymmetriezentren, so können diese unabhängig voneinander sowohl S als auch R konfiguriert sein. Die Verbindungen können als optische Isomere, als Diastereomere, als Racemate oder als Gemische derselben vorliegen.

Die bezeichneten Alkyl- und Perfluoralkylreste können sowohl geradkettig als auch verzweigt vorliegen.

Die Erfindung betrifft weiterhin ein Verfahren zur Herstellung der Verbindungen 1, dadurch gekennzeichnet, daß man Verbindungen
der Formel II 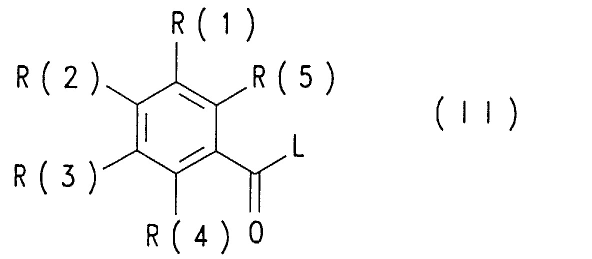 worin R(1) bis R(5) die oben angegebenen Bedeutungen haben und L für eine leicht nucleophil substituierbare Fluchtgruppe,
die ausgewählt ist aus der Gruppe bestehend aus Methoxy, Phenoxy,

Phenylthio, Methylthio, 2-Pyridylthio, Imidazolyl, -OCOOEthyl, -OSO₂-Ethyl,
-O-C( = N-Cyclohexyl)-(NH-Cyclohexyl), -O-N = C[C≡N][(C = O)-OEthyl], steht, mit Guanidin umsetzt.

Die aktivierten Säurederivate der Formel II, worin L eine Alkoxy-, vorzugsweise eine Methoxygruppe oder Phenoxygruppe, eine Phenylthio-, Methylthio-, 2-Pyridylthiogruppe, oder einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.
Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol (L = 1-Imidazolyl, Staab, Angew. Chem. Int. Ed. Engl. 1,351-367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)-methylen)amino]-1,1,3,3-tetramethyluronium-tetrafluoroborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der allgemeinen Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF zwischen 20°C und Siedetemperatur dieser Lösungsmittel bewährt. Bei den meisten Umsetzungen von Verbindungen II mit salzfreien Guanidin wurde vorteilhaft in inerten Lösungsmitteln wie THF, Dimethoxyethan, Dioxan oder Isopropanol gearbeitet. Aber auch Wasser kann als Lösungsmittel dienen.

Wenn L = Cl bedeutet, arbeitet man vorteilhaft unter Zusatz eines Säurefängers, z.B. in Form von überschüssigen Guanidin zur Abbindung der Halogenwasserstoffsäure.

Die unbekannten Verbindungen der Formel II können nach literatur-bekannten Methoden hergestellt werden, indem man beispielsweise 4-Halogen-3-chlorsulfonylbenzoesäuren mit Ammoniak oder Aminen in 3-Aminosulfonyl-4-Halogen-Benzoesäuren bzw. mit einem schwachen Reduktionsmittel wie Natriumbisulfit und anschließende Alkylierung in 3-Alkylsulfonyl-4-Halogen-Benzoesäuren überführt und nach einer der oben beschriebenen Verfahrensvarianten zu erfindungsgemäßen Verbindungen I umsetzt.

Die Einführung der im Phenylteil mit Schwefel-, Sauerstoff- oder Stickstoffnucleophilen substituierten Phenylalanin-Derivate gelingt durch literaturbekannte Methoden der nucleophilen Substitution am Aromaten. Als Abgangsgruppe am Benzoesäurederivat haben sich bei dieser Substitution Halogenide und Trifluormethansulfonate bewährt. Man arbeitet vorteilhaft in einem dipolar aprotischen Lösungsmittel, wie DMF oder TMU, bei einer Temperatur von 0°C bis zum Siedepunkt des Lösungsmittels, bevorzugt von 80°C bis zum Siedepunkt des Lösungsmittels. Als Säurefänger dient vorteilhaft ein Alkali- oder Erdalkalisalz mit einem Anion hoher Basizität und geringer Nucleophilie, zum Beispiel K₂CO₃ oder CsCO₃. Zum Schutz der funktionellen Gruppen der Aminosäure können die bekannten Standardverfahren gewählt werden. Bewährt hat sich am Stickstoff t-Butoxycarbonyl, Benzyloxycarbonyl, Dibenzyl sowie Trityl. An der Säurefunktion kann ohne Schutz gearbeitet werden, oder es kann ein geeigneter Ester oder ein geeignetes Amid Verwendung finden.

Die Einführung der Alkyl- oder Arylsubstituenten gelingt durch literaturbekannte Methoden des Palladium-vermittelten cross-couplings von Arylhalogeniden mit beispielsweise Organozinkverbindungen, Organostannanen, Organoboronsäuren oder Organoboranen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 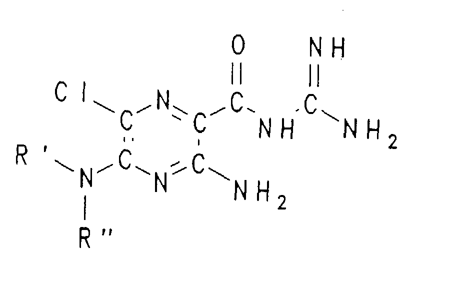 Amilorid: R',R" = H
Dimethylamilorid: R',R" = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen [Circulation 79, 1257-63 (1989)]. Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl. 1): 167 (1988) (book of abstracts)]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 sind Benzoylguanidine beschrieben, die in der den Resten R(1), R(4) und R(5) entsprechenden Stellung ein Wasserstoff-Atom tragen. In der europäischen Offenlegunsgschrift 0 556 674 (HOE 92/F 034) werden Benzoylguanidine beschrieben, in welchen aber die Substituenten nicht die nach der vorliegenden Erfindung beanspruchten Bedeutungen haben. Es sind keine Derivate von Aminosäuren beschrieben. Außerdem läßt die Wasserlöslichkeit dieser bekannten Benzoylguanidine zu wünschen übrig.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Aus den Patent-Publikationen EP 627 413, EP 556 672, EP 589 336, DE 35 02 629 und CA 2 099 445 sind verwandte Benzoylguanidine bekannt; jedoch legt keine dieser Publikationen eine Substitution durch einen Hydroxycarbonsäure- oder Aminosäure-Substituenten nahe oder nimmt sie vorweg.

Es war überraschend, daß die erfindungsgemäßen Verbindungen keine unerwünschten und nachteiligen salidiuretischen, jedoch sehr gute antiarrhythmische Eigenschaften aufweisen, die zur Behandlung von Krankheiten wichtig sind, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.v.-Applikation geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg Körpergewicht, vorzugsweise mindestens 0,01 mg/kg Körpergewicht, bis höchstens 10 mg/kg Körpergewicht, vorzugsweise bis höchstens 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 100 mg pro Tag notwendig werden.

### Liste der Abkürzungen:

- MeOH: Methanol
- DMF: N,N-Dimethylformamid
- TMU: N,N,N',N'-Tetramethylharnstoff
- NBS: N-Bromsuccinimid
- AIBN: α,α-Azo-bis-isobutyronitril
- EI: electron impact
- DCI: Desorption-Chemical Ionisation
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardement
- CH₂Cl₂: Dichlormethan
- THF: Tetrahydrofuran
- eq: Äquivalent
- ES: Elektrospray-Ionisation
- Me: Methyl
- Et: Ethyl
- Bn: Benzyl
- ZNS: Zentralnervensystem
- Brine: gesättigte wäßrige NaCI-Lösung

### Experimenteller Teil

### Allgemeine Vorschrift zur Herstellung von Benzoyl-guanidinen (I)

### Variante A: aus Benzoesäuren (II, L = OH)

0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (Rotationsverdampfer) ab, versetzt mit Wasser, stellt mit 2N HCl auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I)

### Variante B: aus Benzoesäure-alkylestern (II, L = O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

Beispiel 1: N-t-Butoxycarbonyl-4-[(4-guanidinocarbonyl-2-methylsulfonyl) phenoxy]-phenylalanin 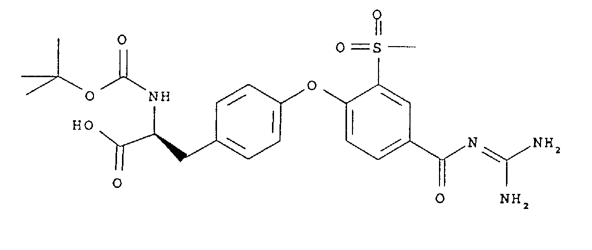 1.8 g N-t-Butoxycarbonyl-4-[(4-methoxycarbonyl-2-methylsulfonyl)phenoxy]-phenylalanin und 1.1 g Guanidin werden nach Variante B zu 700 mg eines farblosen Feststoffs umgesetzt.
mp > 270 °C
R_{f} (Aceton / Wasser 10 : 1) = 0.37 MS (FAB) : 521 (M+H)⁺
a) N-t-Butoxycarbonyl-4-[(4-methoxycarbonyl-2-methylsulfonyl)phenoxy]-phenylalanin
   4.5 g N-t-Butoxycarbonyl-4-[(4-methoxycarbonyl-2-methylsulfonyl)phenoxy]-phenylalanin-benzylester und 500 mg 10% Pd/C werden in 50 ml MeOH unter Normaldruck-Wasserstoff 20 h lang hydriert. Anschließend wird der Katalysator abfiltriert, das Solvens im Vakuum entfernt und mit EE / MeOH 10 : 1 chromatographiert. Man erhält 2.1 g eines farblosen zähen Öls.
   R_{f} (EE / MeOH 10 :1) = 0.12 MS (DCI) : 494 (M+H)⁺
b) N-t-Butoxycarbonyl-4-([4-methoxycarbonyl-2-methylsulfonyl)phenoxy]-phenylalanin-benzylester
   2.8 g N-t-Butoxycarbonyl-tyrosin-benzylester, 2.2 g 4-Fluor-3-trifluormethyl-benzoesäuremethylester und 4.0 g K₂CO₃ werden in 100 ml DMF (wasserfrei) 45 min bei 110 °C gerührt. Das Reaktionsgemisch wird auf 500 ml Wasser gegossen, mit NaHSO₄ auf pH = 2 gestellt und 3 x mit je 200 ml EE extrahiert. Über Na₂SO₄ wird getrocknet und das Solvens im Vakuum entfernt. 4.2 g farbloses Öl.
   R_{f} (DIP) = 0.12 MS (ES) : 584 (M + H)⁺

### Beispiel 2: 4-[(4-Guanidinocarbonyl-2-methylsulfonyl)phenoxy]phenylalanin

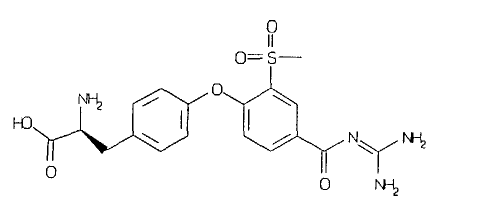

170 mg der Titelverbindung des Beispiels 1 werden in 5 ml CH₂Cl₂ suspendiert, und dazu werden bei RT 61 µl Trifluormethansulfonsäure zugegeben. 90 min wird bei dieser Temperatur gerührt und anschließend auf 50 ml gesättigte wäßrige K₂HPO4-Lösung gegossen. Danach wird 3 mal 100 ml EE extrahiert, über Na₂SO₄ getrocknet und das Solvens im Vakuum entfernt. Man erhält 125 mg eines amorphen Feststoffs.
Rf (EE/MeOH 1:1)=0.43 MS (ES) : 421 (M + H)⁺

### Beispiel 3: N-t-Butoxycarbonyl-4-[(4-guanidinocarbonyl-2-trifluormethyl)phenoxy] phenylalanin

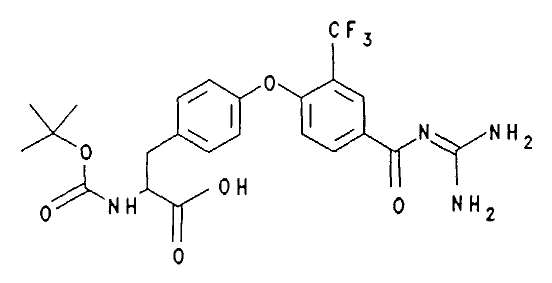

3.5 g N-t-Butoxycarbonyl-4-[(4-methoxycarbonyl-2-trifluormethyl)phenoxy] phenylalanin werden mit 2.2 g Guanidin in 10 ml Isopropanol nach Variante B guanyliert. Man erhält 1.68 g eines farblosen Feststoffs,
mp 231°C.
R_{f} (Aceton/Wasser 20:1) = 0.30 MS (FAB) : 512 (M + H)⁺
a) N-t-Butoxycarbonyl-4-[(4-methoxycarbonyl-2-trifluormethyl)phenoxy] phenylalanin
   4.6 g N-t-Butoxycarbonyl-4-[(4-methoxycarbonyl-2-trifluormethyl)phenoxy] phenylalanin-benzylester und 860 mg 10% Pd auf Aktivkohle (50% Wassergehalt) werden bei Normaldruck und RT 24 h in 100 ml MeOH unter H₂ gerührt. Anschließend wird der Katalysator abfiltriert und die flüchtigen Bestandteile im Vakuum entfernt.
   Man erhält 3.5 g eines farblosen Öls.
   R_{f}(EE/MeOH 10:1) = 0.10 MS (ES) : 484 (M + H)⁺
b) N-t-Butoxycarbonyl-4-[(4-methoxycarbonyl-2-trifluormethyl)phenoxy]phenyl alanin-benzylester
   4.0 g Boc-Tyr-OBn, 2.4 g 4-Fluor-3-trifluormethyl-benzoesäuremethylester und 7.0 g Cs₂CO₃ werden in 30 ml wasserfreiem Tetramethylharnstoff 2 h bei 110°C gerührt. Man läßt abkühlen, verdünnt mit 800 Im EE und wäscht 3 x mit 100 ml Wasser und 3 x mit 100 ml gesättigter wäßriger NaCl-Lösung. Die organische Phase wird über Na₂SO₄ getrocknet, und die Lösungsmittel werden im Vakuum entfernt. Chromatographie an Kieselgel mit DIP liefert 4.6 g eines farblosen Öls.
   R_{f} (DIP) = 0.41 MS (FAB) : 574 (M + H)⁺

### Beispiel 4: 4-[(4-Guanidinocarbonyl-2-trifluormethyl)phenoxy]phenylalanin, Dihydrochlorid

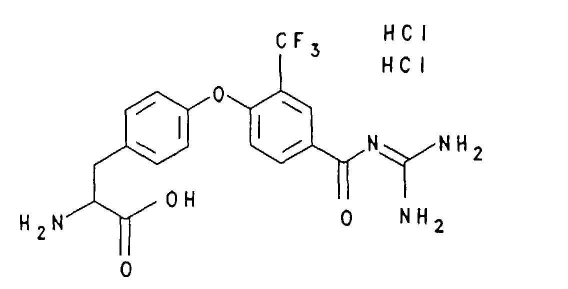

0.8 g N-t-Butoxycarbonyl-4[(4-guanidinocarbonyl-2-trifluormethyl)phenoxy] phenylalanin werden in 50 ml CH₂Cl₂ gelöst und bei 0°C mit 277 µl Trifluormethansulfonsäure versetzt. Man läßt auf RT kommen, dabei fällt ein klumpiger Niederschlag an. Man verdünnt mit 50 ml DME, anschließend wird 1 h bei RT gerührt. Die Lösungsmittel werden im Vakuum entfernt, der Rückstand wird mit 15 ml Wasser aufgenommen und mit gesättigter wäßriger NaHCO₃-Lösung auf pH = 7 eingestellt. Der dabei anfallende Niederschlag wird abfiltriert und an Kieselgel mit CH₂Cl₂/MeOH/H₂O/HOAc 8 : 4 : 1 : 1 chromatographiert. Das Produkt wird in 2 ml 4N HCl gelöst, und die flüchtigen Bestandteile werden im Vakuum entfernt. Man erhält 100 mg farbloser Kristalle,
mp 245°C.
R_{f} ( CH₂Cl₂/MeOH/H₂O/HOAc 8:4:1:1) = 0.27 MS (FAB) : 411 (M + H)⁺

### Pharmakologische Daten:

### Inhibition des Na⁺/H⁺ -Exchangers von Kaninchenerythrocyten

Weiße Neuseeland-Kaninchen (Ivanovas) erhielten eine Standard-Diät mit 2% Cholesterin für sechs Wochen, um den Na⁺/H⁺ -Austausch zu aktivieren und so den Na⁺-Influx in die Erythrocyten via Na⁺/H⁺-Austausch
flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-lnflux zu bestimmen, wurden 100 µl jeder Blutprobe in jeweils 5 ml eines hyperosmolaren Salz-Sucrose-Mediums (mmol/l: 140 NaCI, 3 KCI, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-lnflux ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺-Exchangers: | |
|---|---|
| Beispiel | IC₅₀ µmol/l |
| 1 | 0.43 |
| 2 | 1.0 |
| 4 | 0.026 |

## Patentansprüche

1. Verbindung der Formel I 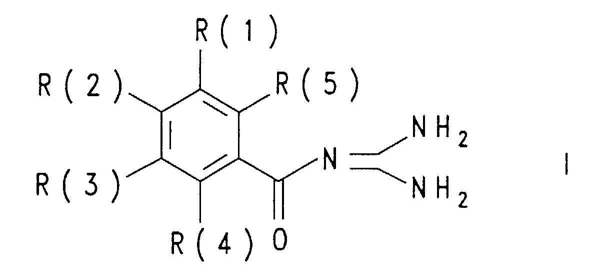 worin bedeuten:
einer der drei Substituenten R(1), R(2) und R(3)
-Y-4-[(CH₂)ₖ-CHR(7)-(C = O)R(8)]-Phenyl,
-Y-3-[(CH₂)ₖ-CHR(7)-(C = O)R(8)]-Phenyl oder
-Y-2-[(CH₂)ₖ-CHR(7)-(C = O)R(8)]-Phenyl,
wobei das Phenyl jeweils unsubstituiert oder substituiert ist mit 1 - 2 Substituenten aus der Gruppe F, Cl, -CF₃, Methyl, Hydroxy, Methoxy, oder -NR(37)R(38);
R(37) und R(38)
unabhängig voneinander Wasserstoff oder -CH₃;
Y eine Bindung, Sauerstoff, -S- oder -NR(9);
R(9) Wasserstoff oder -(C₁ -C₄)-Alkyl;
R(7) -OR(10) oder -NR(10)R(11);
R(10) und R(11)
unabhängig voneinander Wasserstoff, -(C₁-C₈)-Alkyl, -(C₁-C₈)-Alkanoyl, -(C₁-C₈)-Alkoxycarbonyl, Benzyl, Benzyloxycarbonyl;
oder
R(10) Trityl ;
R(8) -OR(12) oder -NR(12)R(13);
R(12) und R(13)
unabhängig voneinander Wasserstoff, -(C₁-C₈)-Alkyl oder Benzyl;
k Null, 1, 2, 3 oder 4;
und die jeweils anderen Reste R(1), R(2) und R(3)
unabhängig voneinander -(C₁-C₈)-Alkyl, -(C₂-C₈)-Alkenyl oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F, Cl, -CF₃, Methyl, Methoxy und -NR(15)R(16);
R(15) und R(16)
Wasserstoff oder -CH₃;
oder die jeweils anderen Reste R(1), R(2) und R(3)
unabhängig voneinander R(18)R(19)N-(C = Y')-NH-SO₂-;
Y' Sauerstoff, -S- oder -N-R(20);
R(18) und R(19)
unabhängig voneinander Wasserstoff, -(C₁-C₈)-Alkyl, -(C₃-C₆)-Alkenyl oder -(CH₂)ₜ-R(21);
t Null, 1, 2, 3 oder 4;
R(21) -(C₅-C₇)-Cycloalkyl oder Phenyl,
welches unsubstituiert ist oder substituiert ist mit 1 - 3 Substituenten ausgewählt aus der Gruppe bestehend aus F, Cl, -CF₃, Methoxy und -(C₁-C₄)-Alkyl;
oder
R(18) und R(19)
gemeinsam 4 oder 5 Methylengruppen, von denen eine CH₂-Gruppe durch Sauerstoff, -S-, -NH-, -N-CH₃ oder -N-Benzyl ersetzt sein kann;
R(20)
wie R(18) definiert oder Amidin; oder
die jeweils anderen Reste R(1), R(2) und R(3)
unabhängig voneinander Wasserstoff, F, Cl, Br, I, -C≡N, X-(CH₂)ₚ-(C_{q}F_{2q + 1}), R(22)-SOᵤ-, R(23)R(24)N-CO-, R(25)-CO- oder R(26)R(27)N-SO₂-, wobei die Perfluoralkylgruppe geradkettig oder verzweigt ist;
X eine Bindung, Sauerstoff, -S- oder -NR(28);
u Null, 1 oder 2;
p Null, 1 oder 2;
q 1, 2, 3, 4, 5 oder 6;
R(22), R(23), R(25) und R(26)
unabhängig voneinander -(C₁-C₈)-Alkyl, -(C₃-C₆)-Alkenyl, -(CH₂)ₙ-R(29) oder -CF₃;
n Null, 1, 2, 3 oder 4;
R(28) Wasserstoff oder -(C₁-C₃)-Alkyl;
R(29) -(C₃-C₇)-Cycloalkyl oder Phenyl,
welches nicht substituiert ist oder substituiert mit 1 - 3 Substituenten ausgewählt aus der Gruppe bestehend aus F, Cl, -CF₃, Methyl, Methoxy und -NR(30)R(31); R(30) und R(31)
Wasserstoff oder -(C₁-C₄)-Alkyl;
oder
R(23), R(25) und R(26)
Wasserstoff;
R(24) und R(27)
unabhängig voneinander Wasserstoff oder -(C₁-C₄)-Alkyl; oder
R(23) und R(24) sowie R(26) und R(27)
gemeinsam 4 oder 5 Methylengruppen, von denen eine CH₂-Gruppe durch Sauerstoff, -S-, -NH-, - N-CH₃ oder -N-Benzyl ersetzt sein kann;
oder
die jeweils anderen Reste R(1), R(2) und R(3)
unabhängig voneinander -OR(35) oder -NR(35)R(36);
R(35) und R(36)
unabhängig voneinander Wasserstoff oder -(C₁-C₆)-Alkyl; oder
R(35) und R(36)
gemeinsam 4 - 7 Methylengruppen, von denen eine CH₂-Gruppe durch Sauerstoff, -S-, -NH-, -N-CH₃ oder -N-Benzyl ersetzt sein kann;
R(4) und R(5)
unabhängig voneinander Wasserstoff, -(C₁-C₄)-Alkyl, F, Cl, -OR(32), -NR(33)R(34) oder -CᵣF₂ᵣ₊₁;
R(32), R(33) und R(34)
unabhängig voneinander Wasserstoff oder -(C₁-C₃)-Alkyl;
r 1, 2, 3 oder 4;
sowie deren pharmazeutisch verträgliche Salze.

2. Verbindung der Formel I nach Anspruch 1, worin bedeuten:
R(1) -(C₁-C₄)-Alkyl, -(C₂-C₄)-Alkenyl oder -(CH₂)ₘR(14);
m Null, 1 oder 2;
R(14) -(C₅-C₆)-Cycloalkyl oder Phenyl,
welches nicht substituiert oder substituiert ist mit 1 - 2 Substituenten ausgewählt aus der Gruppe bestehend aus F, Cl, -CF₃, Methyl, Methoxy und -NR(15)R(16);
R(15) und R(16)
unabhängig voneinander Wasserstoff oder -CH₃;
oder
R(1) R(18)R(19)N-(C = Y')-NH-SO₂-;
Y' Sauerstoff, -S- oder -N-R(20);
R(18) und R(19)
unabhängig voneinander Wasserstoff, -(C₁-C₄)-Alkyl, -(C₃-C₄)-Alkenyl oder -(CH₂)ₜ-R(21);
t Null, 1 oder 2;
R(21)
-(C₅-C₆)-Cycloalkyl oder Phenyl, welches unsubstituiert ist oder substituiert ist mit 1 - 2 Substituenten ausgewählt aus der Gruppe bestehend aus F, Cl, -CF₃, Methoxy und Methyl; oder
R(18) und R(19)
gemeinsam 4 oder 5 Methylengruppen, von denen eine CH₂-Gruppe durch Sauerstoff, -S-, -NH-, -N-CH₃ oder -N-Benzyl ersetzt sein kann;
R(20) wie R(18) definiert oder Amidin;
oder
R(1) Wasserstoff, F, Cl, Br, I, -C≡N, R(22)-SO₂-, R(23)R(24)N-CO-, R(25)-CO- oder
R(26)R(27)N-SO₂-;
R(22), R(23), R(25) und R(26)
unabhängig voneinander -(C₁-C₄)-Alkyl, (C₃-C₄)-Alkenyl, -(CH₂)ₙ-R(29) oder -CF₃;
n Null, 1 oder 2;
R(29) -(C₅-C₆)-Cycloalkyl oder Phenyl,
welches nicht substituiert ist oder substituiert mit 1 - 2 Substituenten ausgewählt aus der Gruppe bestehend aus F, Cl, - CF₃, Methyl, Methoxy und -NR(30)R(31); R(30) und R(31)
unabhängig voneinander Wasserstoff oder Methyl;
oder
R(23), R(25) und R(26)
Wasserstoff;
R(24) und R(27)
unabhängig voneinander Wasserstoff oder Methyl; oder
R(23) und R(24) sowie R(26) und R(27)
gemeinsam 4 oder 5 Methylengruppen, von denen eine CH₂-Gruppe
durch Sauerstoff, -S-, -NH-, -N-CH₃ oder -N-Benzyl ersetzt sein kann; oder
R(1) -OR(35) oder -NR(35)R(36);
R(35) und R(36)
unabhängig voneinander Wasserstoff oder -(C₁-C₄)-Alkyl; oder
R(35) und R(36)
gemeinsam 4 - 5 Methylengruppen, von denen eine CH₂-Gruppe durch Sauerstoff, -S-, -NH-, -N-CH₃ oder -N-Benzyl ersetzt sein kann;
einer der Substituenten R(2) oder R(3)
-Y-4-[(CH₂)ₖ-CHR(7)-(C = O)R(8)]-Phenyl,
-Y-3-[(CH₂)ₖ-CHR(7)-(C = O)R(8)]-Phenyl oder
-Y-2-[(CH₂)ₖ-CHR(7)-(C = O)R(8)]-Phenyl,
wobei das Phenyl jeweils unsubstituiert oder substituiert ist mit 1 - 2 Substituenten aus der Gruppe bestehend aus F, Cl, -CF₃, Methyl, Hydroxy, Methoxy, oder -NR(37)R(38);
R(37) und R(38)
unabhängig voneinander Wasserstoff oder -CH₃;
Y eine Bindung, Sauerstoff, -S- oder -NR(9);
R(9) Wasserstoff oder Methyl;
R(7) -OR(10) oder -NR(10)R(11);
R(10) und R(11)
unabhängig voneinander Wasserstoff, -(C₁-C₅)-Alkyl, -(C₁-C₅)-Alkanoyl, -(C₁-C₄)-Alkoxycarbonyl, Benzyl oder Benzyloxycarbonyl; oder
R(10) Trityl;
R(8) -OR(12) oder -NR(12)R(13) ;
R(12) und R(13)
unabhängig voneinander Wasserstoff, -(C₁-C₄)-Alkyl oder Benzyl;
k Null, 1 oder 2;
und der jeweils andere Substituent R(2) und R(3)
unabhängig voneinander -(C₁-C₄)-Alkyl, Wasserstoff, F, Cl, Br oder I; R(4) und R(5)
unabhängig voneinander Wasserstoff, Methyl, F, Cl, -OR(32), -NR(33)R(34) oder -CF₃;
R(32), R(33) und R(34)
unabhängig voneinander Wasserstoff oder Methyl.

3. Verbindung der Formel I nach einem oder mehreren der Ansprüche 1 bis 2, worin bedeuten:
R(1) -(C₁-C₄)-Alkyl, -(C₂-C₄)-Alkenyl oder -(CH₂)ₘR(14);
m Null, 1 oder 2;
R(14) -(C₅-C₆)-Cycloalkyl oder Phenyl,
welches nicht substituiert oder substituiert ist mit 1 - 2 Substituenten ausgewählt aus der Gruppe bestehend aus F, Cl, - CF₃, Methyl, Methoxy und -NR(15)R(16);
R(15) und R(16)
unabhängig voneinander Wasserstoff oder -CH₃;
oder
R(1) Wasserstoff, F, Cl, Br, I, -C≡N, R(22)-SO₂-, R(23)R(24)N-CO-, R(25)-CO- oder
R(26)R(27)N-SO₂-;
R(22), R(23), R(25) und R(26)
unabhängig voneinander Methyl oder -CF₃,
oder
R(23), R(25) und R(26)
Wasserstoff;
R(24) und R(27)
unabhängig voneinander Wasserstoff oder Methyl;
oder
R(1) -OR(35) oder -NR(35)R(36);
R(35) und R(36)
unabhängig voneinander Wasserstoff oder -(C₁-C₄)-Alkyl;
oder
R(35) und R(36)
gemeinsam 4 - 5 Methylengruppen, von denen eine CH₂-Gruppe durch Sauerstoff, -S-, -NH-, -N-CH₃ oder -N-Benzyl ersetzt sein kann;
einer der Substituenten R(2) und R(3)
-Y-4-[(CH₂)ₖ-CHR(7)-(C = O)R(8)]-Phenyl,
-Y-3-[(CH₂)ₖ-CHR(7)-(C = O)R(8)]-Phenyl oder
-Y-2-[(CH₂)ₖ-CHR(7)-(C = O)R(8)]-Phenyl,
wobei das Phenyl jeweils unsubstituiert oder substituiert ist mit einem Substituenten ausgewählt aus der Gruppe bestehend aus F, Cl, -CF₃, Methyl, Hydroxy, Methoxy und -NR(37)R(38);
R(37) und R(38)
unabhängig voneinander Wasserstoff oder -CH₃;
Y eine Bindung, Sauerstoff, -S- oder -NR(9);
R(9) Wasserstoff oder Methyl;
R(7) -NR(10)R(11);
R(10) und R(11)
unabhängig voneinander Wasserstoff, -(C₁-C₄)-Alkyl, -(C₁-C₅)-Alkanoyl, -(C₁-C₄)-Alkoxycarbonyl, Benzyl oder Benzyloxycarbonyl;
oder
R(10) Trityl;
R(8) -OR(12) oder -NR(12)R(13);
R(12) und R(13)
unabhängig voneinander Wasserstoff, -(C₁-C₄)-Alkyl oder Benzyl;
und der jeweils andere Substituent R(2) und R(3)
unabhängig voneinander -(C₁-C₄)-Alkyl, Wasserstoff, F oder Cl;
R(4) und R(5)
unabhängig voneinander Wasserstoff, Methyl, F, Cl, -OR(32), -NR(33)R(34) oder -CF₃;
R(32), R(33) und R(34)
unabhängig voneinander Wasserstoff oder Methyl.

4. Verfahren zur Herstellung einer Verbindung der Formel I gemäß Anspruch 1, dadurch gekennzeichnet, daß man eine Verbindung der Formel II 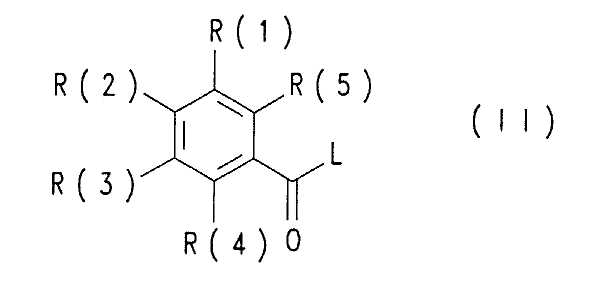 worin R(1) bis R(5) die in Anspruch 1 angegebenen Bedeutungen haben und worin L für eine leicht nucleophil substituierbare Fluchtgruppe steht,
die ausgewählt ist aus der Gruppe bestehend aus Methoxy, Phenoxy,
Phenylthio, Methylthio, 2-Pyridylthio, Imidazolyl, -OCOOEthyl, -OSO₂-Ethyl,
-O-C(= N-Cyclohexyl)-(NH-Cyclohexyl), -O-N = C[C≡N][(C = O)-OEthyl], mit Guanidin umsetzt.

5. Verwendung einer Verbindung I nach Anspruch 1 zur Herstellung eines Medikaments zur Behandlung von Arrhythmien.

6. Verwendung einer Verbindung I nach Anspruch 1 zur Herstellung eines Medikaments zur Behandlung der Angina Pectoris.

7. Verwendung einer Verbindung 1 nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung i nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch 1 zur Herstellung eines Medikaments zur Behandlung von Krankheiten, bei denen die Zellproliferation eine primäre oder sekundäre Ursache darstellt.

16. Heilmittel, enthaltend eine wirksame Menge einer Verbindung I nach einem oder mehreren der Ansprüche 1 bis 3.

## Claims

1. A compound of the formula I 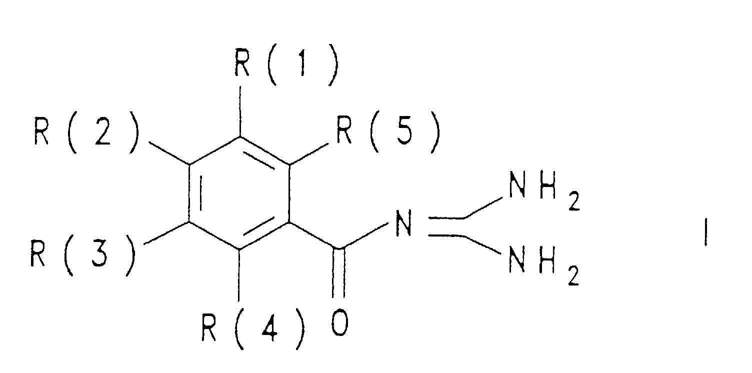 in which:
one of the three substituents R(1), R(2) and R(3) is
-Y-4-[(CH₂)ₖ-CHR(7)-(C=O)R(8)]-phenyl,
-Y-3-[(CH₂)ₖ-CHR(7)-(C=O)R(8)]-phenyl or
-Y-2-[(CH₂)ₖ-CHR(7)-(C=O)R(8)]-phenyl,
in which the phenyl is in each case unsubstituted or substituted by 1 - 2 substituents from the group consisting of F, Cl, -CF₃, methyl, hydroxyl, methoxy and -NR(37)R(38);
R(37) and R(38) independently of one another are
hydrogen or -CH₃;
Y is a bond, oxygen, -S- or -NR(9);
R(9) is hydrogen or -(C₁-C₄)-alkyl;
R(7) is -OR(10) or -NR(10)R(11);
R(10)and R(11) independently of one another are
hydrogen, -(C₁-C₈)-alkyl, -(C₁-C₈)-alkanoyl, - (C₁-C₈) -alkoxycarbonyl, benzyl, benzyloxycarbonyl ; or
R(10) is trityl;
R(8) is -OR(12) or -NR(12)R(13);
R(12) and R(13) independently of one another are
hydrogen, -(C₁-C₈)-alkyl or benzyl;
K is zero, 1, 2, 3 or 4;
and the other particular radicals R(1), R(2) and R(3) independently of one another are
- (C₁-C₈)-alkyl,- (C₂-C₈)-alkenyl or
-(CH₂)ₘR-(14);
m is zero, 1 or 2;
R(14) is -(C₃-C₈)-cycloalkyl or phenyl,
which is unsubstituted or substituted by 1 - 3 substituents chosen from the group consisting of F, Cl, -CF₃, methyl, methoxy and -NR(15)R(16);
R(15) and R(16) are
hydrogen or -CH₃;
or
the other particular radicals R(1), R(2) and R(3) independently of one another are
R(18)R(19)N- (C=Y') -NH-SO₂-;
Y' is oxygen, -S- or -N-R(20);
R(18) and R(19) independently of one another are hydrogen, - (C₁-C₈)-alkyl, - (C₃-C₆)-alkenyl or -(CH₂)ₜ-R(21);
t is zero, 1, 2, 3 or 4;
R(21) is- (C₅-C₇) -cycloalkyl or phenyl,
which is unsubstituted or substituted by 1 - 3 substituents chosen from the group consisting of F, Cl, -CF₃, methoxy and -(C₁-C₄)-alkyl;
or
R(18) and R(19)
together are 4 or 5 methylene groups, one CH₂ group of which can be replaced by oxygen, -S-, -NH-, -N-CH₃ or -N-benzyl;
R(20)
is as defined for R(18) or amidine;
or
the other particular radicals R(1), R(2) and R(3) independently of one another are
hydrogen, F, Cl, Br, I, -CN, X- (CH₂)ₚ-(C_{q}F_{2q+1}), R(22)-SOᵤ-, R(23)R(24)N-CO-, R(25)-CO- or R(26)R(27)N-SO₂-, in which the perfluoroalkyl group is straight-chain or branched;
X is a bond, oxygen, -S- or -NR(28);
u is zero, 1 or 2;
p is zero, 1 or 2;
q is 1, 2, 3, 4, 5 or 6;
R(22), R(23), R(25) and R(26) independently of one another are
- (C₁-C₈) -alkyl,- (C₃-C₆) -alkenyl, - (CH₂)ₙ-R(29) or -CF₃;
n is zero, 1, 2, 3 or 4;
R(28) is hydrogen or -(C₁-C₃)-alkyl;
R(29) is-(C₃-C₇) -cycloalkyl or phenyl,
which is unsubstituted or substituted by 1 - 3 substituents chosen from the group consisting of F, Cl, -CF₃, methyl, methoxy and -NR(30)R-(31);
R(30) and R(31) are
hydrogen or -(C₁-C₄)-alkyl;
or
R(23), R(25) and R(26) are
hydrogen;
R(24) and R(27) independently of one another
are hydrogen or (C₁-C₄)-alkyl;
or
R(23) and R(24), and R(26) and R(27),
together are 4 or 5 methylene groups, one CH₂ group of which can be replaced by oxygen, -S-,-NH-, -N-CH₃ or -N-benzyl;
or
the other particular radicals R(1), R(2) and R(3) independently of one another are
-OR(35) or -NR(35)R(36);
R(35) and R(36) independently of one another are hydrogen or -(C₁-C₆)-alkyl;
or
R(35) and R(36)
together are 4 - 7 methylene groups, one CH₂ group of which can be replaced by oxygen, -S-, -NH-, -N-CH₃ or -N-benzyl;
R(4) and R(5) independently of one another are hydrogen, -(C₁-C₄)-alkyl, F, Cl, -OR(32),
-NR(33)R(34)or -CᵣF₂ᵣ₊₁;
R(32),R(33)and R(34) independently of one another are
hydrogen or -(C₁-C₃)-alkyl;
r is 1, 2, 3 or 4;
or a pharmaceutically tolerated salt thereof.

2. A compound of the formula I as claimed in claim 1, in which:
R(1) is -(C₁-C₄)-alkyl,-(C₂-C₄)-alkenyl or
-(CH₂)-ₘR(14);
m is zero, 1 or 2;
R(14) is (C₅-C₆)-cycloalkyl or phenyl,
which is unsubstituted or substituted by 1 - 2 substituents chosen from the group consisting of F, Cl, -CF₃, methyl, methoxy and -NR(15)R(16);
R(15) and R(16) independently of one another are
hydrogen or -CH₃;
or
R(1) is R(18)R(19)N-(C-Y')-NH-SO₂-;
Y' is oxygen, -S- or -N-R(20);
R(18) and R(19) independently of one another are hydrogen, - (C₁-C₄)-alkyl, - (C₃-C₄)-alkenyl or
-(CH₂)ₜ-R(21);
t is zero, 1 or 2;
R(21) is
-(C₅-C₆)-cycloalkyl or phenyl,
which is unsubstituted or substituted by 1 - 2 substituents chosen from the group consisting of F, Cl, -CF₃, methoxy and methyl;
or
R(18) and R(19)
together are 4 or 5 methylene groups, one CH₂ group of which can be replaced by oxygen, -S-, -NH-, -N-CH₃ or -N-benzyl;
R(20) is as defined for R(18) or amidine;
or
R (1) is hydrogen, F, Cl, Br, I, -CN, R (22) - SO₂-,
R(23)R(24)N-CO-, R(25)-CO- or R(26)R(27)N-SO₂-;
R(22), R(23), R(25) and R(26) independently of one another are
-(C₁-C₄)-alkyl, (C₃-C₄) -alkenyl, -(CH₂)ₙ-R(29) or -CF₃;
n is zero, 1 or 2;
R(29) is -(C₅-C₆)-cycloalkyl or phenyl,
which is unsubstituted or substituted by 1 - 2 substituents chosen from the group consisting of F, Cl, -CF₃, methyl, methoxy and -NR(30)-R(31);
R(30) and R(31) independently of one another are
hydrogen or methyl;
or
R(23), R(25) and R(26) are
hydrogen;
R(24) and R(27) independently of one another are
hydrogen or methyl;
or
R(23) and (R24), and R(26) and R(27),
together are 4 or 5 methylene groups, one CH₂ group of which can be replaced by oxygen, -S-, -NH-, -N-CH₃ or -N-benzyl;
or
R(1) is -OR(35) or -NR(35)R(36);
R(35) and R(36) independently of one another are
hydrogen or -(C₁-C₄)-alkyl;
or
R(35) and R(36)
together are 4 - 5 methylene groups, one CH₂ group of which can be replaced by oxygen, -S-, -NH-, -N-CH₃ or -N-benzyl;
one of the substituents R(2) or R(3) is
-Y-4-[(CH₂)ₖ-CHR(7)-(C=O)R(8)]-phenyl,
-Y-3-[(CH₂)ₖ-CHR(7)-(C=O)R(8)]-phenyl or
-Y-2-[(CH₂)ₖ-CHR(7)-(C=O)R(8)]-phenyl,
in which the phenyl is in each case unsubstituted or substituted by 1 - 2 substituents from the group consisting of F, Cl, -CF₃, methyl, hydroxyl, methoxy or -NR(37)R(38);
R(37) and R(38) independently of one another are
hydrogen or -CH₃;
Y is a bond, oxygen, -S- or -NR(9);
R(9) is hydrogen or methyl;
R(7) is -OR(10) or -NR(10)R(11);
R(10) and R(11) independently of one another are
hydrogen, -(C₁-C₅)-alkyl,-(C₁-C₅)-alkanoyl,-(C₁-C₄)-alkoxycarbonyl, benzyl or benzyloxycarbonyl;
or
R(10) is trityl;
R(8) is -OR(12) or -NR(12)R(13);
R(12) and R(13) independently of one another are
hydrogen,-(C₁-C₄)-alkyl or benzyl;
k is zero, 1 or 2;
and the other particular substituent R(2) or R(3) independently is
-(C₁-C₄)-alkyl, hydrogen, F, Cl, Br or I;
R(4) and R(5) independently of one another are hydrogen, methyl, F, Cl, -OR(32), -NR(33)R(34) or -CF₃;
R(32),R(33)and R(34) independently of one another are
hydrogen or methyl.

3. A compound of the formula I as claimed in one or more of claims 1 to 2, in which:
R(1) is -(C₁-C₄)-alkyl, (C₂-C₄)-alkenyl or -(CH₂)ₘR(14);
m is zero, 1 or 2;
R(14) is -(C₅-C₆)-cycloalkyl or phenyl,
which is unsubstituted or substituted by 1 - 2 substituents chosen from the group consisting of F, Cl, -CF₃, methyl, methoxy and -NR(15)R(16);
R(15) and R(16) independently of one another are
hydrogen or -CH₃;
or
R(1) is hydrogen, F, Cl, Br, I, -CN, R (22) - SO₂-,
R(23)R(24)N-CO-, R(25)-CO- or
R(26)-R(27)-N-SO₂-;
R(22), R(23), R(25) and R(26)independently of one another are
methyl or -CF₃,
or
R(23), R(25) and R(26)are
hydrogen;
R(24) and R(27) independently of one another are
hydrogen or methyl;
or
R(1) is -OR(35) or -NR(35)R(36);
R(35) and R(36) independently of one another are
hydrogen or -(C₁-C₄)-alkyl;
or
R(35) and R(36)
together are 4 - 5 methylene groups, one CH₂ group of which can be replaced by oxygen, -S-, -NH-, -N-CH₃ or -N-benzyl;
one of the substituents R(2) and R(3) is
-Y-4-[(CH₂)ₖ-CHR (7)-(C=O)R(8)]-phenyl,
-Y-3-[(CH₂)ₖ-CHR (7)-(C=O)R(8)]-phenyl or
-Y-2-[(CH₂)ₖ-CHR(7)-(C=O)R(8)]-phenyl,
in which the phenyl is in each case unsubstituted or substituted by one substituent chosen from the group consisting of F, Cl, -CF₃, methyl, hydroxyl, methoxy and -NR(37)R(38);
R(37) and R(38) independently of one another are
hydrogen or -CH₃;
Y is a bond, oxygen, -S- or -NR(9);
R(9) is hydrogen or methyl;
R(7) is -NR(10)R(11);
R(10) and R(11) independently of one another are
hydrogen, -(C₁-C₄)-alkyl,-(C₁-C₅)-alkanoyl, -(C₁-C₄)-alkoxycarbonyl, benzyl or benzyloxycarbonyl;
or
R(10)is trityl;
R(8) is -OR(12) or -NR(12)R(13);
R(12) and R(13) independently of one another are
hydrogen, -(C₁-C₄)-alkyl or benzyl;
and the other particular substituent R(2) or R(3) independently is
-(C₁-C₄)-alkyl, hydrogen, F or Cl;
R(4) and R(5) independently of one another are hydrogen, methyl, F, Cl, -OR(32), -NR(33)R(34) or -CF₃;
R(32),R(33)and R(34) independently of one another are
hydrogen or methyl.

4. A process for the preparation of a compound of the formula I as claimed in claim 1, which comprises reacting a compound of the formula II 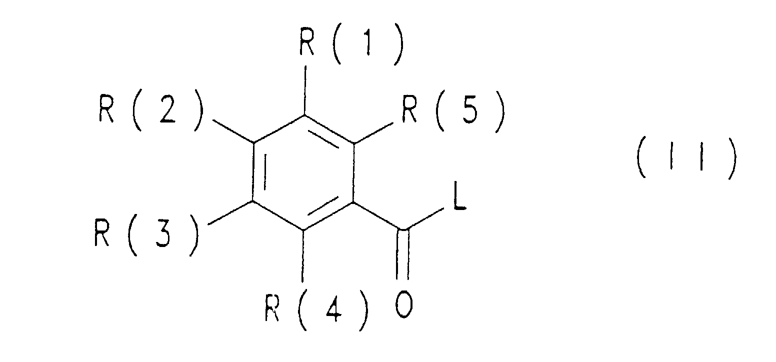 in which R(1) to R(5) have the meanings given in claim 1 and in which L is a leaving group which can easily be substituted nucleophilically,
which is chosen from the group consisting of methoxy, phenoxy, phenylthio, methylthio, 2-pyrdiylthio, imidazolyl, -OCOOethyl, -OSO₂-ethyl, -O-C(=N-cyclohexyl)-(NH-cyclohexyl), O-N=C[C≡N][(C=O)-Oethyl],
with guanidine.

5. The use of a compound I as claimed in claim 1 for the preparation of a medicament for the treatment of arrhythmias.

6. The use of a compound I as claimed in claim 1 for the preparation of a medicament for the treatment of angina pectoris.

7. The use of a compound I as claimed in claim 1 for the preparation of a medicament for the treatment or prophylaxis of cardiac infarction.

8. The use of a compound I as claimed in claim 1 for the preparation of a medicament for the treatment or prophylaxis of angina pectoris.

9. The use of a compound I as claimed in claim 1 for the preparation of a medicament for the treatment or prophylaxis of ischemic states of the heart.

10. The use of a compound I as claimed in claim 1 for the preparation of a medicament for the treatment or prophylaxis of ischemic states of the peripheral and central nervous system and of apoplexy.

11. The use of a compound I as claimed in claim 1 for the preparation of a medicament for the treatment or prophylaxis of ischemic states of peripheral organs and limbs.

12. The use of a compound I as claimed in claim 1 for the preparation of a medicament for the treatment of states of shock.

13. The use of a compound I as claimed in claim 1 for the preparation of a medicament for use during surgical operations and organ transplants.

14. The use of a compound I as claimed in claim 1 for the preparation of a medicament for preserving and storing transplants for surgical measures.

15. The use of a compound I as claimed in claim 1 for the preparation of a medicament for the treatment of diseases where cell proliferation is a primary or secondary cause.

16. A medicine comprising an active amount of a compound I as claimed in one or more of claims 1 to 3.

## Revendications

1. Composé de formule I : 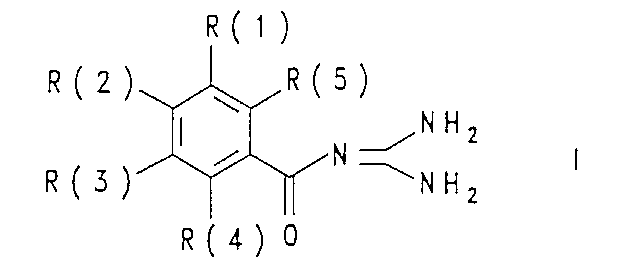 dans laquelle :
un des trois substituants R(1), R(2) et R(3) désigne les groupes :
-Y-4-[(CH₂)ₖ-CHR(7)-(C=O)R(8)]-phényle,
-Y-3-[(CH₂)ₖ-CHR(7)-(C=O)R(8)]-phényle, or
-Y-2-[(CH₂)ₖ-CHR(7)-(C=O)R(8)]-phényle,
où le groupe phényle est respectivement non substitué ou substitué par 1 à 2 substituants choisis parmi les groupes F, Cl, -CF₃, méthyle, hydroxy, méthoxy or -NR(37)R(38) ;
R(37) et R(38) désignent, indépendamment l'un de l'autre, de l'hydrogène ou le groupe -CH₃;
Y est une liaison, de l'oxygène, -S- ou -NR(9) ;
R(9) désigne de l'hydrogène ou un groupe alkyle en C₁-C₄;
R(7) désigne un groupe -OR(10) ou -NR(10)R(11);
où R(10) et R(11) désignent, indépendamment de l'autre, de l'hydrogène, un groupe alkyle en C₁-C₈, alcanoyle en C₁-C₈, alcoxycarbonyle en C₁-C₈, benzyle, benzyloxycarbonyle; or
R(10) est un groupe trityle;
R(8) est un groupe -OR(12) ou -NR(12)R(13);
où R(12) et R(13) désignent, indépendamment l'un de l'autre, de l'hydrogène, un groupe alkyle en C₁-C₈ ou un groupe benzyle;
k est égal à 0, 1, 2, 3 ou 4;
et les autres radicaux respectifs R(1), R(2) et R(3) désignent, indépendamment l'un de l'autre, un groupe alkyle en C₁-C₈, alcényle en C₂-C₈ ou -(CH₂)ₘR(14);
où m est égal à 0, 1 ou 2;
R(14) est un groupe cycloaklyle en C₃-C₈ ou phényle, qui n'est pas substitué ou substitué par 1 à 3 substituants choisis parmi les groupes F, CI, -CF₃, méthyle, méthoxy et -NR(15)R(16);
où R(15) et R(16) désignent de l'hydrogène ou un groupe -CH₃,
ou
les autres radicaux respectifs R(1), R(2) et R(3) désignent, indépendamment l'un de l'autre, le groupe R(18)R(19)N-(C=Y')-NH-SO₂-;
où Y' désigne de l'oxygène , -S- ou le groupe -N-R(20);
R(18) et R(19) désignent, indépendamment l'un de l'autre, de l'hydrogène, un groupe alkyle en C₁-C₈, alcényle en C₃-C₆ ou -(CH₂)ₜ-R(21);
où t est égal à 0, 1, 2, 3 ou 4;
R(21) désigne un groupe cycloalkyle en C₅-C₇ or phényle, qui est non substitué ou substitué par 1 à 3 substituants choisis parmi les groupes F, CI, -CF₃, méthoxy et alkyle en C₁-C₄;
or
(R18) et R(19) désignent conjointement 4 ou 5 groupes méthylène, dont un groupe CH₂ peut être remplacé par de l'oxygène ou les groupes -S-, -NH-, -N-CH₃ ou -N-benzyle;
R(20) est défini comme R(18) ou est une amidine;
ou
les autres radicaux respectifs R(1), R(2) et R(3) désignent, indépendamment l'un de l'autre, de l'hydrogène ou un groupe F, CI, Br, I, -C≡N, X-(CH₂)ₚ-(C_{q}F_{2q+1}), R(22)-SOᵤ-, R(23)R(24)N-CO-, R(25)-CO- ou R(26)R(27)N-SO₂-, le groupe perfluorolkyle étant à chaîne droite ou ramifiée;
X est une liaison, de l'oxygène, -S- ou -NR(28) ;
u est égal à 0, 1 ou 2;
p est égal à 0, 1 ou 2
q est égal à 1, 2, 3, 4, 5 ou 6.
R(22), R(23), R(25) et R(26) désignent, indépendamment l'un de l'autre, un groupe alkyle en C₁-C₈, alcényle en C₃-C₆, -(CH₂)ₙ-R(29) ou -CF₃;
où n est égal à 0, 1, 2, 3 ou 4;
R(28) désigne de l'hydrogène ou un groupe alkyle en C₁-C₃;
R(29) désigne un groupe cycloalkyle en C₃-C₇ ou phényle, qui n'est pas substitut ou est substitué par 1 à 3 substituants choisis parmi les groupes F, CI, -CF₃, méthyle, méthoxy et -NR(30)R(31);
R(30) et R(31) désignent de l'hydrogène ou un groupe alkyle en C₁-C₄;
ou
R(23), R(25) et R(26) désignent de l'hydrogène;
R(24) et R(27) désignent, indépendamment l'un de l'autre, de l'hydrogène ou un groupe alkyle en C₁-C₄;
ou
R(23) et R(24) ainsi que R(26) et R(27) désignent conjointement 4 ou 5 groupes méthylène, dont un groupe CH₂ peut être remplacé par de l'oxygène ou un groupe -S-, -NH-, -N-CH₃ ou -N-benzyle;
ou
les autres radicaux respectifs R(1), R(2) et R(3) désignent, indépendamment l'un de l'autre, un groupe -OR(35) ou -NR(35)R(36) ;
où R(35) et R(36) désignent, indépendamment l'un de l'autre, de l'hydrogène ou un groupe alkyle en C₁-C₆; ou
R(35) et R(36) désignent conjointement 4 à 7 groupes méthylène dont un groupe CH₂ peut être remplacé par de l'oxygène ou un groupe -S-, -NH-, -N-CH₃ ou -N-benzyle; R(4) et R(5) désignent, indépendamment l'un de l'autre, de l'hydrogène, un groupe alkyle en C₁-C₄, F, CI, -OR(32), -NR(33)R(34) ou -CᵣF₂ᵣ₊₁;
où R(32), R(33) et R(34), désignent indépendamment l'un de l'autre, de l'hydrogène ou un groupe alkyle en C₁-C₃;
r est égal à 1, 2, 3 ou 4;
ainsi que leurs sels pharmaceutiquement acceptables.

2. Composé de formule I selon la revendication 1, dans lequel :
R(1) désigne un groupe alkyle en C₁-C₄, alcényle en C₂-C₄ ou -(CH₂)ₘR(14) ;
où m est égal à 0, 1 ou 2;
R(14) est un groupe cycloalkyle en C₅-C₆ ou phényle, qui n'est pas substitué ou est substitué par 1 à 2 substituants choisis parmi les groupes F, CI, -CF₃, méthyle, méthoxy et -NR(15)R(16);
R(15) et R(16) désignent, indépendamment l'un de l'autre, de l'hydrogène ou le groupe -CH₃;
ou
R(1) désigne un groupe R(18)R(19)N-(C=Y')-NH-SO₂-;
où Y' est de l'oxygène, -S- ou -N-R(20);
R(18) et R(19) désignent, indépendamment l'un de l'autre, de l'hydrogène, un groupe alkyle en C₁-C₄, alcényle en C₃-C₄ ou -(CH₂)ₜ-R(21);
où t est égal 0, 1 ou 2;
R(21) désigne un groupe cycloalkyle en C₅-C₆ ou phényle, qui est non substitué ou substitué par 1 ou 2 substituants choisis parmi les groupes comprenant F, CI, CF₃, méthoxy et méthyle;
ou
R(18) et R(19) désignent conjointement 4 ou 5 groupes méthylène, dont un groupe CH₂ peut être remplacé par de l'oxygène ou un groupe -S-, -NH-, -N-CH₃ ou -N-benzyle;
R(20) est défini comme R(18) ou est une amidine;
ou
R(1) désigne de l'hydrogène ou les groupes F, CI, Br, I, - C≡N, R(22)-SO₂-, R(23)R(24)N-CO-, R(25)-CO- ou R(26)R(27)N-SO₄-;
où R(22), R(23), R(25) et R(26) désignent, indépendamment l'un de l'autre, un groupe alkyle en C₁-C₄, alcényle en C₃-C₄, - (CH₂)ₙ-(29) ou -CF₃;
n est égal à 0, 1 ou 2;
R(29) désigne un groupe en C₅-C₆ ou phényle, qui n'est pas substitué ou est substitué par 1 à 2 substituants choisis parmi les groupes F, CI, -CF₃, méthyle, méthoxy et -NR(30)R(31);
où R(30) et R(31) désignent, indépendamment l'un de l'autre, de l'hydrogène ou un groupe méthyle;
ou
R(23), R(25) et R(26) désignent de l'hydrogène;
R(24) et R(27) désignent, indépendamment l'un de l'autre, de l'hydrogène ou un groupe méthyle;
ou
R(23) et R(24) ainsi que R(26) et R(27) désignent conjointement 4 ou 5 groupes méthylène, dont un groupe CH₂ peut être remplacé par de l'oxygène ou un groupe -S-, -NH-, -N-CH₃ ou -N-benzyle;
ou
R(1) désigne un groupe -OR(35) ou -NR(35)R(36);
où R(35) et R(36) désignent, indépendamment l'un de l'autre, de l'hydrogène ou un groupe alkyle en C₁-C₄; ou
R(35) et R(36) désignent conjointement 4 à 5 groupes méthylène, dont un groupe CH₂ peut être remplacé par de l'oxygène ou un groupe -S-, -NH-, -N-CH₃ ou -N-benzyle;
un des substituants R(2) ou R(3) désigne un groupe
-Y-4-[(CH₂)ₖ-CHR (7)-(C=O)R(8)]-phényle,
-Y-3-[(CH₂)ₖ-CHR (7)-(C=O)R(8)]-phényle, ou
-Y-2-[(CH₂)ₖ-CHR(7)-(C=O)R(8)] phényle,
où le groupe phényle est respectivement non substitué ou substitué par 1 à 2 substituants choisis parmi les groupes F CI, -CF₃, méthyle, hydroxy, méthoxy ou -NR(37)R(38);
où R(37) et R(38) désignent, indépendamment l'un de l'autre, de l'hydrogène ou le groupe -CH₃;
Y est une liaison, de l'oxygène, -S- ou -NR(9);
où R(9) désigne de l'hydrogène ou un groupe méthyle;
R(7) désigne un groupe -OR(10) ou -NR(10)R(11);
où R(10) et R(11) désignent, indépendamment l'un de l'autre, de l'hydrogène, un groupe alkyle en C₁-C₅, alcanoyle en C₁-C₄, alcoxycarbonyle en C₁-C₄, benzyle ou benzyloxycarbonyle;
ou
R(10) est un groupe trityle;
R(8) est un groupe -OR(12) ou -NR(12)R(13);
où R(12) et R(13) désignent, indépendamment l'un de l'autre, de l'hydrogène, un groupe alkyle en C₁-C₄ ou benzyle;
k est égal à 0, 1, 2;
et les autres substituants respectifs R(2) et R(3) désignent, indépendamment l'un de l'autre, un groupe alkyle en C₁-C₄), de l'hydrogène, F, CI, Brou I;
R(4) et R(5) désignent, indépendamment l'un de l'autre, de l'hydrogène, un groupe méthyle, F, CI, -OR(32), -NR(33) R(34) ou -CF₃;
où R(32), R(33) et R(34) désignent, indépendamment l'un de l'autre, de l'hydrogène ou un groupe méthyle.

3. Composé de formule I selon une ou plusieurs des revendications 1 et 2, dans lequel :
R(1) désigne un groupe alkyle en C₁-C₄, alcényle en C₂-C₄ ou -(CH₂)ₘR(14) ;
où m est égal à 0, 1 ou 2;
R(14) est un groupe cycloalkyle en C₅-C₆ ou phényle, qui n'est pas substitué ou est substitué par 1 à 2 substituants sélectionnés parmi les groupes F, CI, -CF₃, méthyle, méthoxy et -NR(15)R(16);
où R(15) et R(16) désignent, indépendamment l'un de l'autre, de l'hydrogène ou le groupe -CH₃;
ou
R(1) désigne de l'hydrogène ou un groupe F, CI, Br, I, -C≡N, R(22)-SO₂-, R(23)R(24)N-CO-, R(25)-CO- ou R(26)R(27)-N-SO₂,
où R(22), R(23), R(25) et R(26) désignent, indépendamment l'un de l'autre, un groupe méthyle ou -CF₃,
ou
R(23), R(25) et R(26) désignent de l'hydrogène;
R(24) et R(27) désignent, indépendamment l'un de l'autre, de l'hydrogène ou un groupe méthyle;
ou
R(1) désigne un groupe -OR(35) ou -NR(35)R(36);
où R(35) et R(36) désignent, indépendamment l'un de l'autre, de l'hydrogène ou un groupe alkyle en C₁-C₄; ou
R(35) et R(36) désignent conjointement 4 à 5 groupes méthylène, dont un groupe CH₂ peut être remplacé par de l'oxygène ou un groupe -S-, -NH-, -N-CH₃ ou -N-benzyle;
un des substituants R(2) et R(3) désigne un groupe
-Y-4-[(CH₂)ₖ-CHR (7)-(C=O)R(8)]-phényle,
-Y-3-[(CH₂)ₖ-CHR (7)-(C=O)R(8)]-phényle, ou
-Y-2-[(CH₂)ₖ-CHR(7)-(C=O)R(8)]-phényle ,
où le groupe phényle est respectivement non substitué ou substitué par un substituant choisi parmi les groupes F, CI, -CF₃, méthyle, hydroxy, méthoxy et -NR(37)R(38);
où R(37) et R(38) désignent, indépendamment l'un de l'autre, de l'hydrogène ou le groupe -CH₃;
Y est une liaison, de l'oxygène, -S- ou -NR(9);
R(9) désigne de l'hydrogène ou un groupe méthyle;
R(7) désigne un groupe -NR(10)NR(11);
où R(10) et R(11) désignent, indépendamment de l'autre, de l'hydrogène, un groupe alkyle en C₁-C₄, alcanoyle en C₁-C₅, alcoxycarbonyle en C₁-C₄, benzyle ou benzyloxycarbonyle;
R(10) est un groupe trityle;
R(8) est un groupe -OR(12) ou -NR(12)R(13);
où R(12) et R(13) désignent, indépendamment l'un de l'autre, de l'hydrogène, un groupe alkyle en C₁-C₄ ou benzyle;
et les autres substituants respectifs R(2) et R(3) désignent, indépendamment l'un de l'autre, un groupe alkyle en C₁-C₄, de l'hydrogène, F ou CI;
R(4) et R(5) désignent, indépendamment l'un de l'autre, de l'hydrogène, un groupe méthyle, F, CI, -OR(32), -NR(33) R(34) ou -CF₃;
où R(32), R(33) et R(34) désignent, indépendamment l'un de l'autre, de l'hydrogène ou un groupe méthyle.

4. Procédé de fabrication d'un composé de formule I selon la revendication 1, caractérisé en ce que l'on fait réagir un composé de formule II : 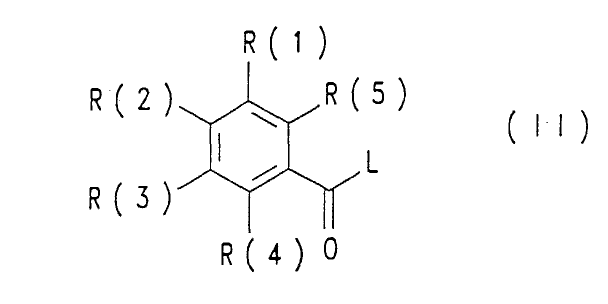 dans laquelle R(1) à R(5) ont les significations indiquées dans la revendication 1 et dans laquelle L désigne un groupe labile aisément éliminable par substitution nucléophile choisi dans le groupe comprenant les groupes méthoxy, phénoxy, phénylthio, méthylthio, 2-pyridylthio, imidazolyle, -OCOO-éthyle, -OSO₂-éthyle, -O-C(=N-cyclohexyl)-(NH-cyclohexyle), -O-N=C[C-N][(C=O)-O-éthyle], avec de la guanidine.

5. Utilisation d'un composé I selon la revendication 1 pour la préparation d'un médicament pour le traitement des arythmies.

6. Utilisation d'un composé I selon la revendication 1 pour la préparation d'un médicament pour le traitement de l'angine de poitrine.

7. Utilisation d'un composé I selon la revendication 1 pour la préparation d'un médicament pour le traitement ou la prophylaxie de l'infarctus du myocarde.

8. Utilisation d'un composé I selon la revendication 1 pour la préparation d'un médicament pour le traitement ou la prophylaxie de l'angine de poitrine.

9. Utilisation d'un composé I selon la revendication 1 pour la préparation d'un médicament pour le traitement ou la prophylaxie d'états ischémiques du coeur.

10. Utilisation d'un composé I selon la revendication 1 pour la préparation d'un médicament pour le traitement ou la prophylaxie d'états ischémiques des systèmes nerveux périphérique et central et l'apoplexie.

11. Utilisation d'un composé I selon la revendication 1 pour la préparation d'un médicament pour le traitement ou la prophylaxie d'états ischémiques d'organes périphériques et des membres.

12. Utilisation d'un composé I selon la revendication 1 pour la préparation d'un médicament pour le traitement d'états de choc.

13. Utilisation d'un composé I selon la revendication 1 pour la préparation d'un médicament susceptible d'être utilisé dans des opérations chirurgicales et des transplantations d'organes.

14. Utilisation d'un composé I selon la revendication 1 pour la préparation d'un médicament pour la conservation et le stockage de transplants pour des opérations chirurgicales.

15. Utilisation d'un composé I selon la revendication 1 pour la préparation d'un médicament pour le traitement de maladies dont la prolifération cellulaire constitue une cause primaire ou secondaire.

16. Médicament contenant une quantité efficace d'un composé I selon une ou plusieurs des revendications 1 à 3.
